# EUROPEAN PATENT APPLICATION

(11) **EP 4 534 548 A1**
(43) Date of publication of application: **09.04.2025**
(21) Application number: 23201917.4
(22) Date of filing: 05.10.2023
(51) Int. Cl.: C07K 14/005, A61K 38/16

(54) **VIRUS-LIKE PARTICLES WITH ENHANCED OPTICAL PROPERTIES**

(71) Applicant: Eberhard Karls Universität Tübingen Medizinische Fakultät, 72074 Tübingen (DE)
(72) Inventor: EBERT, Domenic, 74564 Crailsheim (DE); CONRADY, Marcel-Chris, 55252 Mainz-Kastel (DE); SIMON, Claudia, 72144 Dusslingen (DE)
(74) Representative: Witte, Weller & Partner Patentanwälte mbB

(57) **Abstract**

The present invention relates to a recombinant fusion protein, nucleic acid molecule encoding said recombinant fusion protein, an expression vector comprising said nucleic acid molecule, a biological cell comprising the nucleic acid molecule or the expression vector, a virus-like particle (VLP) comprising a multiple number of the recombinant fusion protein, and to a method of producing the VLP.

## Description

The present invention relates to a recombinant fusion protein, nucleic acid molecule encoding said recombinant fusion protein, an expression vector comprising said nucleic acid molecule, a biological cell comprising the nucleic acid molecule or the expression vector, a virus-like particle (VLP) comprising a multiple number of the recombinant fusion protein, and to a method of producing the VLP.

### FIELD OF THE INVENTION

The present invention relates to the field of recombinant proteins, in particular recombinant virus-derived capsid fusion proteins.

### BACKGROUND OF THE INVENTION

Particles with optical properties are often used in the area of diagnostics and drug delivery, as well as for analytic equipment like fluorescence-activated cell sorting (FACS) or nanoparticle tracking analysis (NTA). The optical property thereby enables the particles to be tracked and detected by means of imaging methods. Often used thereby are so-called fluorophores coupled to said particles, i.e., fluorescent chemical compounds that can emit light when excited by light.

One factor limiting the use of particles with optical properties in the field of medical imaging applications or analytics is the brightness of the particles. This is often too low, for example, because too few color or fluorescent molecules are provided per particle, or fluorophores are quenched due to the close proximity per particle/molecule.

Holmes et al. (2022), Ultrafast Collective Excited-State Dynamics of a Virus-Supported Fluorophore Antenna, J. Phys. Chem. Lett. 13, 14, 3237-3243, describe that immobilization of chemical fluorophores on the bromomosaic virus (BMV) capsid results in increased fluorescence of the fluorophores. However, the increase in fluorescence occurs only up to a critical number of fluorophores per capsid protein or per virus particle, respectively, and therefore cannot be further increased beyond that. With the BMV capsid and the proposed chemical immobilization of the fluorophore the maximal number of fluorophores per molecule is the limiting number to further increase the brightness of the molecule.

One object underlying the present invention is therefore to provide a building block for the production of optical particles, which are useful in medical imaging applications and analytics, where the disadvantages of the prior art are avoided or at least reduced. In particular, such a component is to be provided, with which particles with defined and improved optical properties can be produced, and which are characterized by an increased brightness.

### SUMMARY OF THE INVENTION

This object is met by a recombinant fusion protein comprising an amino acid surface loop of a virus-derived capsid protein, wherein a fluorescent protein (FP) is inserted into said loop via amino acid linkers.

The inventors have realized that engineering a virus-derived capsid protein by grafting a fluorescent protein into a defined surface loop results in a display of the fluorophore in a large, stoichiometrically precise number per capsid and a regular manner. The engineered capsid protein is still able to assemble into virus-like particles (VLP), which are nanoparticles with enhanced optical properties and increased brightness.

According to the invention, a "recombinant protein" is a protein produced biotechnologically, preferably with the aid of genetically modified organisms or with transiently transfected cell cultures or by means of a protein synthesizer.

The recombinant fusion protein according to the invention is preferably provided in isolated form, which means that it is essentially or fully purified from its environment, e.g., the expression system or components thereof, and enriched or provided in a highly pure form.

According to the invention, a "capsid protein" is understood to be a viral protein that forms the capsid or coat of a virus or parts thereof. It is also referred to as 'viral coat protein' (VCP). For the virus, the capsid serves to coat its genetic material. Most capsid proteins accumulate into helical or icosahedral units. An example of a capsid protein particularly suitable according to the invention is the major capsid protein VP1 of polyomavirus.

The capsid protein in the recombinant fusion protein of the invention is "virus-derived", meaning that it originates from a naturally occurring virus or is encoded by a viral gene, but has been technically modified genetically by insertion of the fluorescent protein (FP).

According to the invention, a fluorescent protein (FP) is a protein that exhibits fluorescence when exposed to light in a certain wavelength range. An example of a prominent and well-suited fluorescent protein is the green fluorescent protein (GFP).

According to the invention, a "fusion protein" or chimeric protein is created through the joining of two or more genes that originally code for separate proteins. In the invention, the gene encoding the viral capsid protein is fused with the gene encoding the fluorescent protein. The fusion of the two genes is achieved in such a way that the fluorescent protein is cloned into a surface loop of the viral capsid protein via amino acid linkers located at both ends, i.e., N and C terminally, of the fluorescent protein (FP).

According to the invention, a "surface loop" is understood to be a section in the viral capsid protein that, after complete folding of the capsid protein under physiological conditions and taking the final secondary and tertiary structure, is directed or arranged on the top side of the protein or outwardly into the environment of the protein. For example, at least four suitable surface loops are found in the capsid protein VP1 of murine polyomavirus, where they are designated HI, DE, BC, and FG.

According to the invention, an "amino acid linker" is a short section of amino acids, e.g., 5-10 and preferably 6 or 7 amino acids, that serves to couple or fuse the fluorescent protein to or with the capsid protein in the surface loop. The amino acid linker ensures that the recombinant fusion protein can fold into the correct shape and assemble with further fusion proteins. The amino acid linkers are located at both ends, i.e., N and C terminally, of the fluorescent protein (FP).

The object underlying the invention is herewith fully achieved.

In an embodiment of the invention said capsid protein comprises a jelly roll topology.

The jelly roll or Swiss roll fold is a protein fold or supersecondary structure that is highly conserved among viral proteins, especially viral capsid proteins. It is typically composed of eight beta strands arranged in two four-stranded sheets. By this special protein folding, surface loops are configured that are particularly well suited for cloning the fluorescent protein (FP), ensuring that the fluorescent protein (FP) is exposed on the top or outside of the fusion protein of the invention and provides a strong light signal.

In a further embodiment of the invention said virus-derived capsid protein is a polyomavirus- or a papillomavirus-derived capsid protein.

Polyomaviruses and papillomaviruses, which are structurally very similar, are characterized by capsid proteins, which are particularly well suited for the production of the recombinant fusion protein of the invention. According to the invention, all variants of polyoma and papilloma viruses are included, e.g., murine, human, avian and other species-specificities. For example, the capsid of polyomavirus is formed by the 'major capsid protein' VP1 and the 'minor capsid proteins' VP2 and VP3. The capsid of papillomaviruses is composed of two different capsid proteins, the major capsid protein L1 as well as the minor capsid protein L2. In particular, VP1 and L1 are especially suitable for the production of the recombinant fusion protein according to the invention.

Polyomavirus capsids are larger than, e.g., brome mosaic virus (BMV) capsids (in the murine polyomavirus variant capsids have a diameter of 45 nm vs. a diameter of 28 nm in BMV) and are composed of more copies of capsid protein (360 vs. 180 in BMV).

Compared to prior art virus-like particles (VLPs) (Holmes et al., 2022, op. cit.), those VLPs produced by assembly of the recombinant protein of the invention are characterized by high brightness and by ease of production via cloning of a fluorescent protein into a surface loop of a virus-derived capsid protein. Unlike in the prior art, the recombinant protein according to the invention ensures that every capsid protein displays one fluorophore always at the same position. As a result, the fluorophore is displayed regularly on the surface obeying the icosahedral symmetry of the capsid scaffold.

Therefore, in yet another embodiment of the invention said capsid protein is polyomavirus capsid protein VP1.

Sequence information on the above capsid protein can be obtained from the relevant protein databases, e.g., VP1 (major capsid protein) [murine polyomavirus strain A2] = GenBank AAB59902.1.

In still another embodiment of the invention said surface loop is selected from the group consisting of the following surface loops: HI, DE, BC, and FG.

This measure has the advantage of cloning and insertion of the fluorescent protein into such sections of the viral capsid protein that exposure to the surface or outer surface of the fusion protein of the invention is ensured. This provides a strong fluorescent signal and further allows formation of pentameric subunits of the recombinant protein and assembly into virus-like particles (VLP). The exact location of the respective surface loops is known to the skilled person.

For example, for the murine polyomavirus the exact location of the HI loop of VP1 can be found in Stehle et al. (1994), Structure of murine polyomavirus complexed with an oligosaccharide receptor fragment, Nature 369, 160-163, and Stehle and Harrison 1996), Crystal structures of murine polyomavirus in complex with straight-chain and branched-chain sialyloligosaccharide receptor fragments, Structure 4(2), 183-194. The HI loop of the L1 capsid protein in papillomaviruses is, e.g., described in Viscidi et al. (2023), Bioengineered Bovine Papillomavirus L1 Protein Virus-like Particle (VLP) Vaccines for Enhanced Induction of CD8 T Cell Responses through Cross-Priming, Int. J. Mol. Sci. 2023, 24(12), 9851. The HI loop of capsid protein VP1 of the adeno-associated virus (AAV) is further described in DiPrimio et al. (2008), Surface Loop Dynamics in Adeno-Associated Virus Capsid Assembly, Journal of Virology 82(11):5178-89. Die HI loop of capsid protein IX of adenovirus (AV) is described in Dmitriev et al. (2002), Engineering of Adenovirus Vectors Containing Heterologous Peptide Sequences in the C Terminus of Capsid Protein IX, Virol. 76(14): 6893-6899.

In an embodiment of the invention the FP is inserted after an asparagin (N) in the VP1 amino acid sequence, which, in the VP1 of murine polyomavirus, small plaque strain 16, is at position 294 (N294), and is inserted before a tyrosine (Y), which is in this strain at position 295 (Y295). The fusion protein according to the invention then comprises the following structure: N-terminal section of capsid protein with HI loop up to N294 - linker - FP - linker - C-terminal section of capsid protein starting with HI loop at Y295 on until C-terminus.

In another embodiment of the invention said fluorescent protein (FP) is a green-fluorescence-protein-like protein (GFP-like protein), preferably selected from the group consisting of: green fluorescent protein (GFP), preferably superfolder GFP (sfGFP), yellow fluorescent protein (YFP), red fluorescent protein (RFP), blue fluorescent protein (BFP).

This measure has the advantage that such fluorescent proteins are employed which have proven useful in cloning practice and are therefore well suited for working the invention. In principle, all GFP-like proteins are suitable according to the invention, since they are structurally very similar. A good overview of these proteins can be found in https://www.fpbase.org/lineage/. The specific FPs listed above are examples of preferred variants that the inventors have found to produce good results.

In a further embodiment of the invention said virus-derived capsid protein is derived from a murine polyomavirus, preferably from the murine polyomavirus small plaque strain 16.

This measure has the advantage that such a capsid protein is used, which the inventors have tested and verified in their experiments, and which is particularly well suited.

In still another embodiment said surface loop of the VP comprises the amino acid sequence of SEQ ID NO: 1 (HI loop).

This measure has the advantage that the structure plan for the HI loop is provided from the VP1 capsid protein of the murine polyomavirus. It is therefore easy for the skilled person to produce the fusion protein of the invention, e.g., by means of recombination biology.

In yet another embodiment of the invention the VP comprises an amino acid sequence of SEQ ID NO:2 (VP1).

With this measure, the amino acid sequence of the entire VP1 capsid protein from murine polyoma virus is provided. This makes it easy for the skilled person to produce in biological expression systems the recombinant fusion protein according to the invention or to synthesize it via protein synthesizers.

In a further embodiment of the invention said FP comprises an amino acid sequence of SEQ ID NO:3 (sfGFP).

By this measure the amino acid sequence of a superfolding variant of GFP (sfGFP) is provided, which is a robust variant designed for *in vivo* high-throughput screening of protein expression levels. It shows increased thermal stability and is able to retain its fluorescence even when fused to poorly folding proteins.

In yet another embodiment of the invention said amino acid linkers comprise an amino acid sequence of GSGSSH (SEQ ID NO:4) and/or GSSGSGS (SEQ ID NO:5), preferably GSGSSH is N-terminally and GSSGSGS is C-terminally located with respect to the FP.

This measure has the advantage that such amino acid linkers are employed, which have been shown by the inventors to be well suited for correctly cloning and orienting the fluorescent protein into the capsid protein. It is ensured that the final recombinant protein can still fold correctly, form pentameric subunits and assemble into a virus-like particle (VLP).

In another embodiment of the invention the recombinant fusion protein comprises the amino acid sequence of SEQ ID NO: 6.

Advantageously, the total amino acid sequence of an embodiment of the essential sections of the recombinant fusion protein is provided hereby.

Another subject-matter of the invention relates to a nucleic acid molecule, preferably an expression vector, encoding the recombinant fusion protein according to the invention.

With this subject invention, a useful tool is provided for the production of the recombinant fusion protein according to the invention. All kinds of nucleic acid molecules, DNA, RNA, etc., as well as all suitable expression vectors, in particular those for an expression of the protein in bacterial cells, such as *E. coli,* consisting of natural or modified nucleotides, are eligible and encompassed. It goes without saying that the nucleic acid molecule may comprise common regulatory elements, such as promoters, enhancers, terminators etc.

The properties, embodiments, advantages and features of the recombinant fusion protein according to the invention apply equally correspondingly to the nucleic acid molecule according to the invention and the expression vector according to the invention.

A further subject-matter of the invention relates to a biological cell comprising the nucleic acid molecule and/or the expression vector according to the invention.

All cells can be taken into consideration as biological cells, such as bacterial (e.g., *E. coli*), mammalian, human and other biological cells.

The properties, embodiments, advantages and features of the recombinant fusion protein according to the invention apply equally correspondingly to the biological cell according to the invention.

A further subject-matter of the present invention is a virus-like particle (VLP) comprising a multiple number of the recombinant fusion protein according to the invention. Preferably, said VLP comprises subunits formed of pentamers of the recombinant fusion protein.

This measure provides a construct that is suitable as a delivery system for therapeutics, genes and other molecules and structures, and can be tracked by means of imaging methods due to its particularly good fluorescent properties.

The properties, embodiments, advantages and features of the recombinant fusion protein according to the invention apply correspondingly to the virus-like particle (VLP) according to the invention.

Another subject-matter of the invention relates to a method of producing a VLP, preferably the VLP according to the invention, comprising (i) the recombinant production of the recombinant fusion protein according to the invention, preferably via expression in a bacterial system, further preferably in *E.coli,* (ii) purifying the recombinant fusion protein, preferably as pentameric subunits, further preferably via chromatography, and (iii) assembling the recombinant protein into a VLP.

It goes without saying that the method is preferably carried out in an appropriate buffer system and under experimental conditions which are well-known to the skilled artisan.

The properties, embodiments, advantages and features of the recombinant fusion protein according to the invention apply correspondingly to method according to the invention.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments and examples resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

Reference is made to figures in which the following is shown.

### BRIEF DESCRIPTION OF THE FIGURES

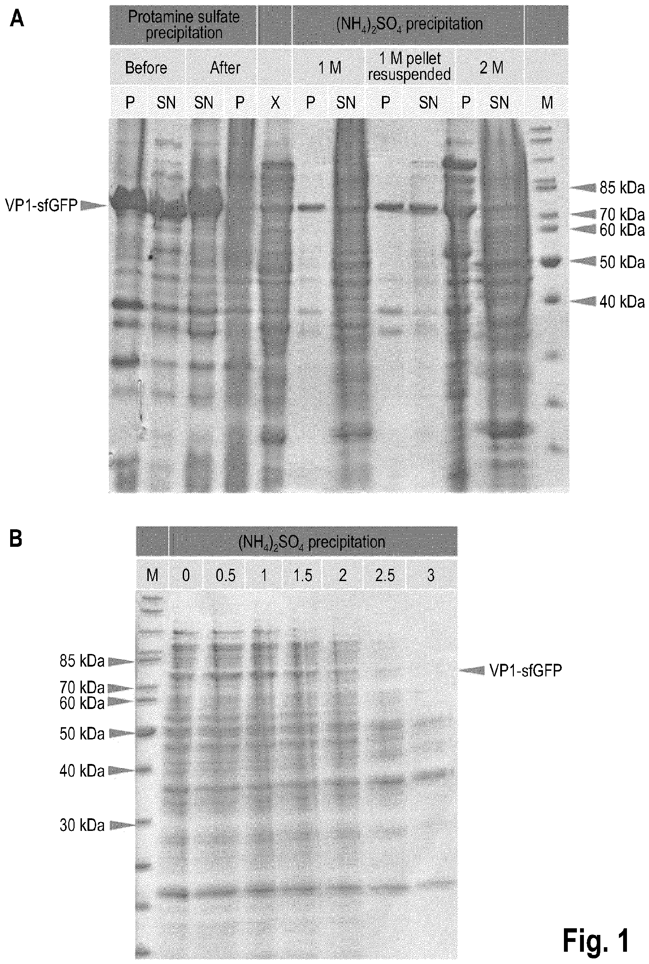

- Figure 1:: Coomassie gels of the VP1-sfGFP purification (M= Marker, SN= Supernatant, P= pellet, X= unknown sample, FT=flow-through, W= wash, E= elution). Gel (A) shows the protein sample before and after protamine sulfate as well as at the different (NH₄)₂SO₄ precipitation steps. After 1 M (NH₄)₂SO₄, the supernatant was used for the 2 M step. The pellet of the 1 M step was resuspended in ZA-buffer to check for loss of the protein of inter-est. Gel (B) show the (NH₄)₂SO₄ dilution row, done to find out which are the right steps for the precipitation to enrich the protein of interest and purify other contaminants out. It was decided to use a 1 M step and a 2 M step of (NH₄)₂SO₄.

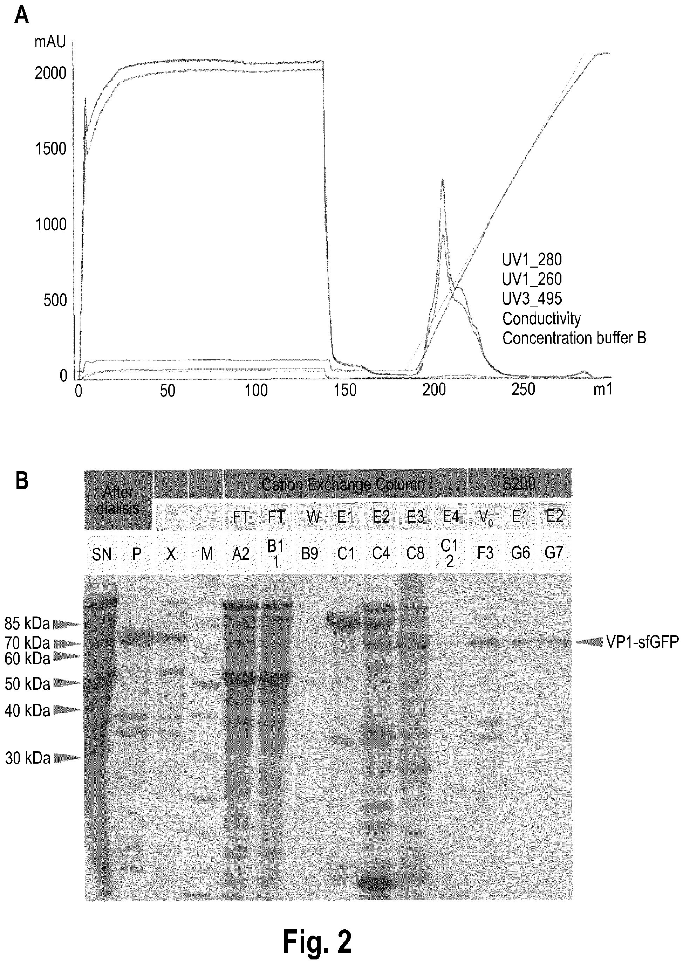

- Figure 2: (A) Ion exchange chromatography ((HighTrap SPHP, 5 mL) of VP1-sfGFP on a Akta purifier. After dialysis against Ared-buffer. The protein sample was diluted 1:15 in lExA-buffer and loaded on a with I ExA-buffer equilibrated cation exchange column (HighTrap SPHP, 5 mL). The binding of the protein sample was performed with a flow rate of 1 mL/min, the elution was done with a flow rate of 2 mL/min with a linear gradient of 25 column volumes from 0% to 100 % buffer B. The VP1-sfGFP pentamers could be pooled at around 30 % I ExB-buffer. It can be seen a small pink peak at that spot which is the signal of the sfGFP bound to VP1. The pentamer peak is a shoulder of a bigger peak which shows another big contaminant protein in the sample. The FT shows a lot of sfGFP absorption of the VP1-sfGFP protein (pink) which shows that the protein of interest does not have a sufficient binding affinity to the column. (B) Coomassie gels of the VP1-sfGFP purification after dialysis against Ared-buffer and the results of the ion exchange chromatography and the following size exclusion chromatography.

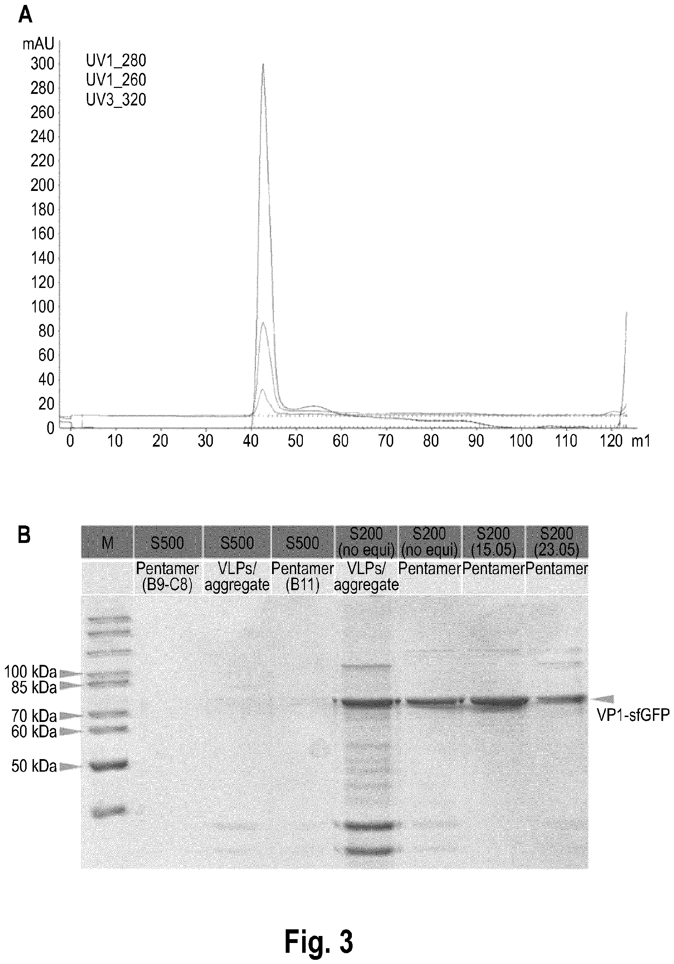

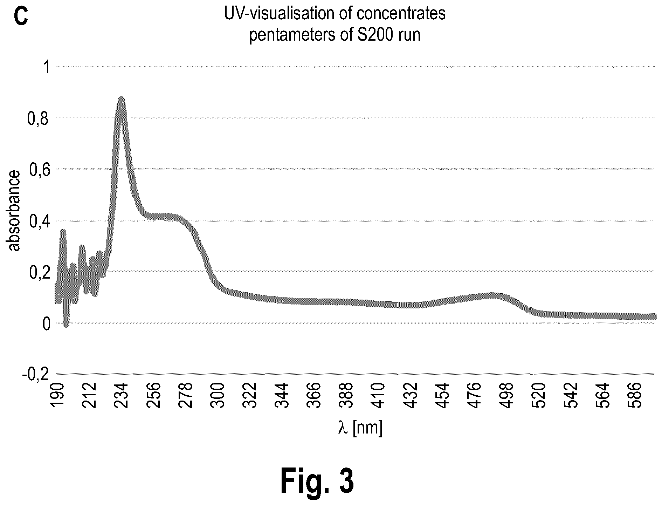

- Figure 3:: (A) Size exclusion chromatography (HiLoad 16/600 Superdex 200 pg) of VP1-sfGFP. The elution of the ion exchange chromatography was centrifuged and resuspended in Ared-buffer and was loaded on a size exclusion column (HiLoad 16/600 Superdex 200 pg) which was equilibrated with Ared-buffer. The VP1-sfGFP pentamers could be pooled at around 55 mL where a big peak is shown. (B) Coomassie gel of the VP1-sfGFP purification after SEC (HiLoad 16/600 Superdex 200 pg and Hiprep 26/60 Sephacryl S500 HR). The gel shows a big loss of the protein of interest after SEC on the S500 compared with the SEC on the S200. Therefore, it was further worked with the pooled pentamer fraction from the S200 run. (C) UV-visualisation of the concentrated VP1-sfGFP pentamers pooled from the S200. The VP1-sfGFP pentamers were concentrated to a concentration of 0.095 mg/mL.

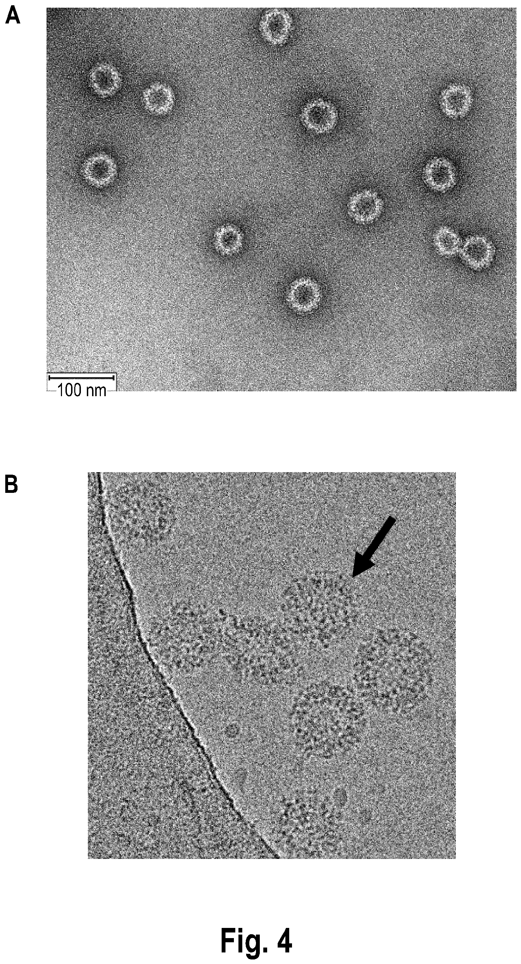

- Figure 4:: Electron microscopy of VP1-sfGFP VLPs (c = 0.2 mg/mL, in A0-buffer). (A) Negative stained transmission electron microscopy image prepared with 1 % uranyl acetate. Examination showed monodisperse VLPs with a diameter of 55 nm and a few smaller VLPs with a diameter of 41 nm. (B) Cryogenic electron microscopy image of VP1-sfGFP VLPs (arrow) shows a homogeneous size and characteristic.

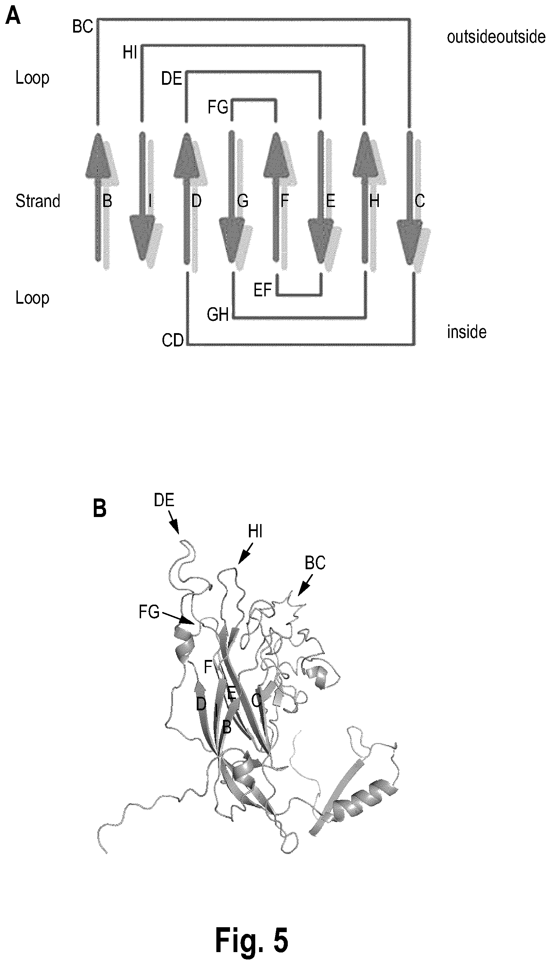

- Figure 5:: Illustration of the formation of loops in the sense of a 'jelly roll topology' schematically in a viral capsid protein (A) and specifically in the viral capsid protein VP1 of the murine polyoma virus (B).

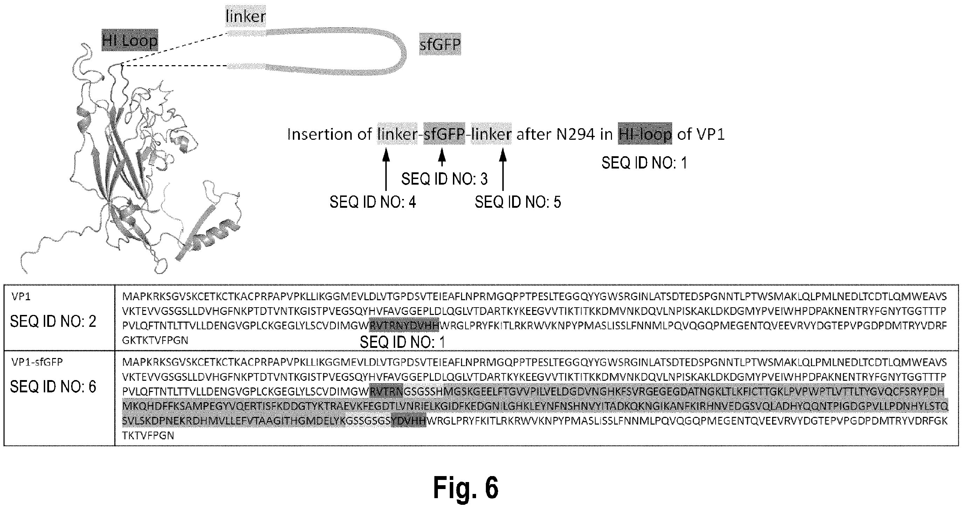

- Figure 6:: Schematic illustration of an embodiment of the VP1-sfGFP fusion protein according to the invention along with amino acid sequences of the individual sections.

### EXAMPLES

### 1. Cloning via Gibson assembly and transformation of BL21 cells

Cloning is carried out by Gibson assembly which is a molecular cloning method developed by Daniel Gibson. Multiple DNA fragments with overlapping ends of at least 20 bp can be assembled in an isothermal reaction. For the reaction three enzymes are required: A 5' exonuclease which digest the 5' end expose the complementary sequences which are required for annealing (sticky ends). After annealing of the DNA fragments a DNA polymerase fills the gaps and a DNA ligase seals the nicks to get a fully covalently linked DNA fragment; see Gibson et al. (2009). Enzymatic assembly of DNA molecules up to several hundred kilobases. Nature methods 6/5, 343-345.

The backbone and insert for the Gibson assembly were produced by PCR and need 20-150 bp sequence homology overhangs. Success of PCR was analyzed by agarose gel electrophoresis. Afterwards, it was performed a Dpn I cleavage with the backbone and insert of the PCR reaction to digest remaining plasmid DNA in the sample. It was mixed 10 µL of backbone or insert with 0.5 µL of Dpn I (New England BioLabs), 2.5 µL CutSmart buffer (New England BioLabs) and 12 µL dH₂O. The samples were incubated for 15 min at 37 °C and the reaction was afterwards stopped by heating the samples for 20 min at 80 °C. A 15 µL aliquot of the Gibson assembly master mix was thawed on ice. Then 0.5 µL of the backbone and 2 µL of each insert was added to the Gibson master mix and the mixture was incubated at 50 °C for 1 h. The whole mixture was used for transformation of BL21 competent *E. coli* cells by methods known in the art.

### 2. Protein purification and assembly of VP1-sfGFP VLPs

After a large-scale expression of VP1-sfGFP in BL21 at 25 °C, 80 g of the wet biomass was lysed and treated as described. A benzonase incubation and protamine sulfate precipitation was done to get rid of DNA, (NH₄)₂SO₄ precipitation was done to enrich the protein of interest missing any affinity tag as can be seen in Fig. 1. Followed by a cation exchange chromatography (HighTrap SPHP, 5 mL) shown in Fig. 2 and final a size exclusion chromatography (HiLoad 16/600 Superdex 200 pg shown in Fig. 3. The VP1-sfGFP pentamers could be pooled, are concentrated up by flow filtration followed by an assembly overnight.

The assembled VP1-sfGFP sample was subsequently loaded either onto an SEC column (SuperoseTM 6 Increase 10/300 GL) or onto a SEC column contain a matrix with a larger pore size (SephacrylTM S-500 HR TricornTM 10/300 GL) for separation of different VLP species.

VLPs run on the SuperoseTM 6 Increase 10/300 GL column at an elution volume of around 9 mL and VP1-sfGFP pentamers at around 15 mL. Assembled VP1-sfGFP VLPs showed an absorption signal of 200 mAU. The analytical SephacrylTM S-500 HR TricornTM 10/300 GL column allows a wider separation due to its matrix with larger pore size which lead to wider peaks (compared to the sharp peaks of the SuperoseTM 6 Increase 10/300 GL column). The VLPs run on the S-500 column at an elution volume of around 15 mL. The peak at around 12 mL is probably a different VLP species. Protein concentration of the purified VLPs was examined by UV-VIS spectroscopy. For the correct calculation of the protein concentration, the light scattering proportion caused by the large size of the VLPs was subtracted from the spectrum as described. A final concentration of 0.23 mg/mL of VP1-sfGFP VLPs was calculated using the absorption at 280 nm and the Lambert-Beer-law with the extinction coefficient ε = 77280 M-1 cm-1, calculated by ProtParam (ExPASy). Therefore, assembly of 2.38 mg VP1-sfGFP pentamers led to a yield of 0.46 mg VP1-sfGFP VLPs.

The morphology of the assembled VP1-sfGFP VLPs was examined by transmission electron microscopy of negative stained samples (Figure 4A) and cryogenic electron microscopy (Figure 4B). The electron microscopy pictures confirm the monodisperse characteristics of the VP1-sfGFP VLPs already shown by dynamic light scattering. Nearly all of the VLPs have a diameter of around 55 nm which is around 10 nm larger than the icosahedral VLPs described in literature caused by the exposed sfGFP on the VLP surface. A few smaller ones with a diameter of around 41 nm probably correspond to octahedral VLPs described in literature with a diameter of 32 nm.

Further experiments were carried out by the inventors by using spinning disk confocal microscopy to investigate fluorescence emission properties of the VP1-sfGFP VLPs. It was found that the VP1-sfGFP VLPs according to the invention have significantly higher brightness than the industrial standard (data not shown).

### 3. Schematic illustration of the fusion protein VP1-sfGFP according to the invention

Fig. 5 illustrates the formation of loops in the sense of a 'jelly roll topology' schematically (A) and specifically on the viral capsid protein VP1 of the murine polyomavirus (B). On the outside of the capsid protein, the loops designated with BC, HI, DE and FG are formed, on the inside the loops designated with CD, GH and EF. This topology is highly conserved among viral capsid proteins.

An embodiment variant of the recombinant VP1-sfGFP fusion protein according to the invention along with amino acid sequences of the individual sections is shown schematically in Fig. 6. The construct "Linker-sfGFP-Linker" is inserted into the HI loop of the VP1 capsid protein of the murine polyomavirus after N294.

### 4. Characterization of stability and biological activity of the VP1-sfGFP VLPs

The chemical and biophysical characterization of the VP1-sfGFP VLPs was performed to examine its stability and its sialic acid binding activity. For the determination of the thermal stability the inventors performed a thermal unfolding transition assay at a pH of 7.4 from 8 °C to 98 °C with the VP1-sfGFP VLPs, pentamer and sfGFP. The inventors measured the thermal stability of the protein and solely of the sfGFP chromophore by the intrinsic fluorescence and the sfGFP fluorescence, respectively.

For the intrinsic fluorescence, the inventors could observe, that VP1-sfGFP VLPs show an onset of unfolding transition at 36 °C, VP1-sfGFP pentamers at 28 °C and sfGFP shows no unfolding transition under given conditions. They are, therefore, thermally stable enough for a clinical usage.

The sfGFP chromophore in VP1-sfGFP VLPs, VP1-sfGFP pentamer and sole sfGFP show a high thermal stability at a physiological pH of 7.4. The inventors could observe an onset of unfolding transition measured by the sfGFP fluorescence for the sole sfGFP at 84 °C. VP1-sfGFP pentamers show a two-step unfolding transition, first at 27 °C which probably is connected with the pentamer unfolding transition previously examine with the intrinsic fluorescence and a second unfolding transition at 84 °C which is the unfolding of the sfGFP chromophore. VP1-sfGFP VLPs showed an onset of sfGFP unfolding at 88 °C.

In their experiments, the inventors could also detect green fluorescent aggregates of VP1-sfGFP, which verifies the fact that still after aggregation of the VP1 scaffold, due to its great stability sfGFP still show fluorescence properties. sfGFP is highly stable and shows fluorescence activity up to temperature above 80 °C.

The inventors further examined the chemical stability of VP1-sfGFP VLPs, pentamer and sole sfGFP with rising concentration of the chaotropic salt Guadinium-HCl and temperatures from 35 °C to 42 °C using the sfGFP fluorescence signal. The inventors could observe that sfGFP is stable until 4.2 M Gua-HCl independent of the temperature. VP1-sfGFP VLPs which showed comparable sfGFP chromophore stability to VP1-sfGFP pentamers, in the thermal stability assay, were chemically stable until a concentration of 1.8 M Gua-HCl whereas the pentamers were stable until 4.2 M Gua-HCl independent of the temperature.

## Claims

1. A recombinant fusion protein comprising an amino acid surface loop of a virus-derived capsid protein, wherein a fluorescent protein (FP) is inserted into said loop via amino acid linkers.

2. The recombinant fusion protein of claim 1, wherein said capsid protein comprises a jelly roll topology.

3. The recombinant fusion protein of claim 1 or 2, wherein said virus-derived capsid protein is a polyomavirus- or a papillomavirus-derived capsid protein.

4. The recombinant fusion protein of any of the preceding claims, wherein said capsid protein is VP1.

5. The recombinant fusion protein of any of the preceding claims, wherein said surface loop is selected from the group consisting of the following surface loops: HI, DE, BC, and FG.

6. The recombinant fusion protein of any of the preceding claims, wherein the fluorescent protein (FP) is selected from the group consisting of: green fluorescent protein (GFP), preferably superfolder GFP (sfGFP), yellow fluorescent protein (YFP), red fluorescent protein (RFP), blue fluorescent protein (BFP).

7. The recombinant fusion protein any of the preceding claims, wherein said virus-derived capsid protein is derived from a murine polyoma virus, preferably from the murine polyoma virus small plaque strain 16.

8. The recombinant fusion protein of any of the preceding claims, wherein said surface loop of the VP comprises the amino acid sequence of SEQ ID NO: 1 (HI loop).

9. The recombinant fusion protein of any of the preceding claims, wherein the VP comprises an amino acid sequence of SEQ ID NO:2 (VP1).

10. The recombinant fusion protein of any of the preceding claims, wherein the FP comprises an amino acid sequence of SEQ ID NO:3 (sfGFP).

11. The recombinant fusion protein of any of the preceding claims, wherein the amino acid linkers comprise an amino acid sequence of GSGSSH (SEQ ID NO:4) and/or GSSGSGS (SEQ ID NO:5), preferably GSGSSH is N-terminally and GSSGSGS is C-terminally located with respect to the FP.

12. The recombinant fusion protein of any of the preceding claims, which comprises the amino acid sequence of SEQ ID NO: 6.

13. A nucleic acid molecule encoding the recombinant fusion protein of any of claims 1-12.

14. A virus-like particle (VLP) comprising a multiple number of the recombinant fusion protein of any of claims 1-12, preferably comprising subunits formed of pentamers of the recombinant fusion protein of any of claims 1-12.

15. A method of producing the VLP of claim 14, comprising (i) the recombinant production of the recombinant fusion protein of any of claims 1-12, preferably via expression in a bacterial system, further preferably in *E.coli,* (ii) purifying the recombinant fusion protein, preferably as pentameric subunits, further preferably via chromatography, and (iii) assembling the recombinant protein into a VLP.
